(19) **Europäisches Patentamt / European Patent Office / Office européen des brevets**

(11) **EP 4 413 919 A1**

(12) **EUROPEAN PATENT APPLICATION**

(43) Date of publication:
**14.08.2024 Bulletin 2024/33**

(21) Application number: **23155623.4**

(22) Date of filing: **08.02.2023**

(51) International Patent Classification (IPC):
**A61B 5/029** (2006.01) **A61B 5/00** (2006.01)

(52) Cooperative Patent Classification (CPC):
**A61B 5/029; A61B 5/022; A61B 5/4872;**
A61B 5/0215; G16H 50/20; G16H 50/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **MUEHLSTEFF, Jens**
  **Eindhoven (NL)**
• **WIJSHOFF, Ralph Wilhelm Christianus Gemma
  Rosa**
  **Eindhoven (NL)**

• **DERKX, Rene Martinus Maria**
  **5656AG Eindhoven (NL)**
• **MENA BENITO, Maria Estrella**
  **Eindhoven (NL)**
• **PELSSERS, Eduard Gerard Marie**
  **Eindhoven (NL)**
• **SCHMITT, Lars**
  **Eindhoven (NL)**
• **DAVIDOIU, Valentina-Geta**
  **Eindhoven (NL)**

(74) Representative: **Philips Intellectual Property &
Standards**
**High Tech Campus 52**
**5656 AG Eindhoven (NL)**

(54) **DEVICE, SYSTEM AND METHOD FOR DETERMINING ONE OR MORE HEMODYNAMIC PARAMETERS OF A SUBJECT**

(57) The present invention relates to a device, system and method for determining one or more hemodynamic parameters of a subject. The method comprises obtaining tissue pressure information indicating tissue pressure of tissue of a body portion of the subject; obtaining subject demographic information; obtaining body-related information related to the subject's body; estimating fat-free mass of the subject from the subject demographic information and the obtained body-related information; and determining the one or more hemodynamic parameters of the subject from the obtained tissue pressure information, the estimated fat-free mass and the subject demographic information. The body-related information indicates one or more of one or more parameters of a hemodynamic measurement apparatus used for measuring tissue pressure and/or one or more other measurement parameters, one or more subject-related parameters or subject-related information different from the subject demographic information, and treatment-related information indicating information about medical treatment of the subject.

FIG.6

**Description**

FIELD OF THE INVENTION

The present invention relates to a device, system and method for determining one or more hemodynamic parameters of a subject, such as an individual or patient.

BACKGROUND OF THE INVENTION

Hemodynamic measurements are used in many medical examinations to assess the condition of a subject. The availability of hemodynamic monitoring (HDM) ensures optimal tissue perfusion and oxygen delivery while maintaining adequate mean arterial blood pressure of the patient. For example, a commonly conducted hemodynamic measurement is the measurement of blood pressure (BP) but may include measurement of other parameters of the functional characteristics of the heart and the circulatory system that affect the perfusion of tissues with oxygenated blood. BP can either be obtained invasively via an arterial blood-pressure catheter (ABP) or non-invasively via a BP inflatable cuff (NIBP).

Continuous advanced hemodynamic monitoring is available only to few patients. ABP allows for direct pressure measurement, blood sampling for hemodynamics monitoring and also medication titration. However, invasive BP monitoring is usually used only in the case of high-risk patients or in complex surgical procedures because it is associated with adverse effects, such as e.g. infections. The large majority of patients are monitored using traditional non-invasive technologies (e.g. NIBP, SpO2, ECG, respiration rate). On the other hand, the conventional NIBP technology supports only BP measurements and does not offer any additional hemodynamic parameter (e.g. cardiac output, stroke volume, etc.).

US 2015/359446 A1 discloses a blood pressure measuring system configured to surround a patient's body part, comprising pressurization means for applying pressure to the body part, and comprising a kinking-proof shell, wherein the kinking-proof shell is arranged so as to be located between the pressurization means and the body part, when the blood pressure measuring system surrounds the body part. Such a blood pressure measuring system is also referred to as Advanced Monitoring Cuff (AMC) or, as used in the following, Shell Cuff (SC) which may be used for hemodynamic measurements.

The SC uses a non-invasive hemodynamic monitoring technology that can measure hemodynamic parameters such as stroke volume (SV) and cardiac output (CO). The accuracy and precision of the SV and CO measurements of the SC is an important aspect on which the SC system is compared to other competitive solutions for HDM.

WO 2019/211210 A1 discloses a method for determining a cardiac stroke volume (SV) using a demographic model, comprising the steps of: providing a first pulse contour stroke volume based on one or more characteristics of a measured arterial blood pressure waveform or providing a conventionally derived pulse contour stroke volume, determining at least one perfusion parameter descriptive for the perfusion through the fat free mass and the adipose mass of a body of the individual, and/or determining at least one fluid responsiveness parameter function depending on a fluid responsiveness parameter descriptive for a heart-lung interaction of the individual, and adjusting the first pulse contour stroke volume or the conventionally derived pulse contour stroke volume based on at the least one of the perfusion parameter and/or the fluid responsiveness parameter function to provide a second pulse contour stroke volume.

SUMMARY OF THE INVENTION

It is an object of the present invention to provide a device, system and method that allow an improved accuracy in the determination of one or more hemodynamic parameters of a subject.

In a first aspect of the present invention a device for determining one or more hemodynamic parameters of a subject is presented the device comprising circuitry configured to:

- obtain tissue pressure information indicating tissue pressure of tissue of a body portion of the subject;
- obtain subject demographic information;
- obtain body-related information related to the subject's body indicating one or more of
- one or more parameters of a hemodynamic measurement apparatus used for measuring tissue pressure and/or one or more other measurement parameters,
- one or more subject-related parameters or subject-related information different from the subject demographic information, and
- treatment-related information indicating information about medical treatment of the subject;
- estimate fat-free mass of the subject from the subject demographic information and the obtained body-related information; and
- determine the one or more hemodynamic parameters of the subject from the obtained tissue pressure information, the estimated fat-free mass and the subject demographic information.

In a further aspect of the present invention a system for determining one or more hemodynamic parameters of a subject is presented comprising:

- an inflatable cuff;
- an inflator configured to inflate the inflatable cuff; and
- a device as disclosed herein.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to overcome shortcomings of known systems by providing a more personalized approach. While the current SC system as well as other systems for HDM make use of demographic models relating to a general population to estimate patient characteristics, according to the present invention estimates of the characteristics of the subject are tailored to the individual subject which leads to HDM parameters which are more accurate for the individual subject. Specifically, the presented approach personalizes the inputs and the processing to the subject, which helps to have a more accurate estimation of fat-free mass (FFM), which consequently leads to a more accurate determination of hemodynamic parameters (herein also called HDM parameters) such as SV and CO. This personalized approach, taking into account specifics of the patient and/or contextual information, leads to a more accurate measurement of one or more hemodynamic parameters over various patient populations.

According to the present invention, not only tissue pressure information (as e.g. measured by a pressure sensor or BP measurement device) and demographic information relating to the subject (as e.g. entered by a user or the subject or as taken from any database holding such demographic information of the subject) are used as input, but additionally body-related information related to the subject's body is used to estimate FFM of the subject and the desired hemodynamic parameter(s). The body-related information is generally a kind of measurement information that relates to the body, e.g. the body composition, of the subject and is useful to better assess the subject's body composition, FFM and body fat, which in turn is useful in computing the desired hemodynamic parameter(s).

According to an embodiment an inflatable cuff and an inflator configured to inflate the inflatable cuff may be used to acquire pieces of information for use by the device and method in the determination of the desired hemodynamic parameter(s). In particular, tissue pressure and actuator pressure (as parameter of a hemodynamic measurement apparatus) may be acquired by the inflatable cuff.

In a preferred embodiment the inflatable cuff is a Shell Cuff (or AMC). Such a Shell Cuff comprises an inflatable bladder (also called actuator) that is wrapped around a (stiff or semi-stiff) shell. Between the shell and the arm, there is a fluid-filled pressure sensor which is connected to a disposable pressure transducer (DPT) which converts the pressure measured by the pressure sensor into an electrical signal that represents the tissue pressure. Because a solid shell surrounds the pressure sensor, the pressure sensor is hydraulically coupled to the tissue of the arm, i.e. the pressure measured by the pressure sensor is referred to as tissue pressure. The pressure measured in the inflatable bladder, which may be an air-filled or a fluid-filled inflatable bladder, represents the actuator pressure. Instead of using the DPT to obtain the tissue pressure, a pressure sensor may be directly placed in the fluid-filled pressure sensor to convert the pressure in the pressure sensor into an electrical signal. Such a pressure sensor could be similar like a Millar Mikro-Tip solid state pressure catheter. This pressure sensor would replace the fluid-filled tube and DPT. Alternatively, the tissue pressure may be measured by placing a piezo film sensor between the tissue and the shell, which would replace the fluid-filled pressure sensor, the fluid-filled tube and the DPT altogether.

For the different pieces of information for use by the present invention multiple different options and combinations may be used. In an embodiment, as parameters of a hemodynamic measurement apparatus used for measuring tissue pressure or as other measurement parameters, one or more of actuator pressure and cuff size may be used. The hemodynamic measurement apparatus may thus be a BP measurement apparatus, e.g. a Shell Cuff.

As subject-related parameters or subject-related information, one or more of the following pieces of information may be used: circumference of the subject's arm, circumference of the subject's waist or abdomen, circumference of the subject's leg, one or more images of the subject's arm, one or more images of the subject's body, triceps skinfold thickness of the subject's arm, near infrared interactance of the subject's arm tissue, one or more medical images of the subject, and genetic information of the subject, urine output of the subject, and amount and/or type of food consumed by the subject. Such information can be acquired in different ways, e.g. by direct measurement at the subject, by an imaging apparatus (e.g. a camera, CT, X-ray, MR, US, DXA, etc.), via input from the subject or a user, from a database or record (e.g. a medical record) holding information regarding the subject, by a weight scale, by a bioimpedance measurement, by determining food intake, etc.

As treatment-related information, one or more of a bioimpedance analysis of the subject's thorax and/or body, administered fluids or medication, length of stay in a hospital, and estimated muscle loss during hospital stay may be used. Such information may be acquired by direct measurement at the subject, by accessing a database or record holding such information, via input from the subject or a user, etc.

As subject demographic information, one or more of height, weight, age and gender of the subject may be used. Such information may be acquired as well by direct measurement at the subject, by accessing a database or record holding such information, via input from the subject or a user, etc.

According to another embodiment the circuitry may be configured to use, as body-related information, an actuator pressure of an actuator used for inflating a cuff of the hemodynamic measurement apparatus; determine a ratio between the actuator pressure and the tissue pressure or between the rates at which the actuator pressure and the tissue pressure increase during inflation of the cuff; and use the determined ratio in the step of estimating the fat-free mass and/or the step of determining the one or more hemodynamic parameters. It has been found that such a ratio represents a good indicator for the FFM and hemodynamic parameters, which can thus be computed with higher accuracy in a scenario where a hemodynamic measurement apparatus is used, such as a Shell Cuff.

The circuitry may further be configured to provide an actuator control signal to the actuator to apply actuator pressure sequences and to obtain actuator pressure values and tissue pressure values while the actuator sequences are applied. These pairs of obtained actuator pressure values and tissue pressure values can be used to determine body-related information (e.g. about the arm's anatomy) and thus the FFM of the subject.

In another embodiment the circuitry is configured to track the subject's weight over time based on one or more of the subject-related parameters, the subject-related information and the treatment-related information and to update the estimation of the fat-free mass in case of a weight change. This further improves the accuracy of the determination and the monitoring of the hemodynamic parameters.

According to an embodiment the circuitry is configured to estimate fat-free mass by use of a regression equation, which may be based on statistics or historical data to define a relationship between FFM and one or more pieces of subject demographic information and body-related information. This provides a simple but effective way of estimating FFM.

Generally, the hemodynamic parameters can be determined using the method disclosed in WO 2019/211210 A1, 2019, e.g. the method described therein in the context of Figs. 1 to 7 of this document, according to which FFM is used to assess stroke volume (SV). The description of the method(s) disclosed in this document is herein incorporated by reference. The present invention provides improvements, particularly with respect to the estimation of FFM, which lead to an improved accuracy and flexibility of the determination of hemodynamic parameter(s).

According to an embodiment the circuitry is configured to determine the one or more hemodynamic parameters by

- determining a hemodynamic status of the subject from the tissue pressure information waveform and the subject demographic information, and
- determining a calibrated estimate of the hemodynamic parameter, in particular the subject's stroke volume, by use of the fat-free mass, in particular by adjustment with a perfusion parameter that includes estimates of the fat-free mass and adipose mass of the subject.

Hereby, a first estimate of a hemodynamic parameter, in particular the subject's stroke volume, may be determined from the hemodynamic status, and the first estimate of the hemodynamic parameter, in particular the subject's stroke volume, may then be calibrated into a second estimate of stroke volume by use of the fat-free mass, in particular by adjustment with a perfusion parameter that includes estimates of the fat-free mass and adipose mass of the subject.

BRIEF DESCRIPTION OF THE DRAWINGS

These and other aspects of the invention will be apparent from and elucidated with reference to the embodiment(s) described hereinafter. In the following drawings

Fig. 1 shows a diagram of the relationship between CO and FFM for several subjects.
Fig. 2 shows a schematic diagram of an embodiment of a system according to the present invention.
Fig. 3 shows a schematic diagram of an embodiment of a Shell Cuff.
Fig. 4 shows a schematic diagram of a first embodiment of a device according to the present invention.

Fig. 5 shows a schematic diagram of a second embodiment of a device according to the present invention.

Fig. 6 shows a schematic diagram of a third embodiment of a device according to the present invention.

Fig. 7 shows a diagram of the ratio between the low-pass filtered actuator pressure and tissue pressure signals for a person having a BMI of 18.

Fig. 8 shows a diagram of the ratio between the low-pass filtered actuator pressure and tissue pressure signals for a person having a BMI of 29.

Fig. 9 shows a flow chart for a first method for improving a first pulse contour stroke volume, PCSV_uncal, by means of a perfusion parameter, BioCal, and to output a second pulse contour stroke volume, PCSV _imp.

Fig. 9A shows a flow chart for determining the first pulse contour stroke volume, PCSV_uncal.

Fig. 9B shows a part of an arterial blood pressure waveform for determining the first pulse contour stroke volume, PCSV_uncal.

Fig. 10 shows a flow chart for determining a perfusion parameter, BioCal, as a part of the first method for improving a first pulse contour stroke volume.

Fig. 11 shows a model for perfusion of adipose mass, AM, and fat free mass, FFM, depending on layer thickness of adipose mass, AM/H, and layer thickness of fat free mass, FFM/H.

Fig. 12 shows examples of relations between a perfusion coefficient of fat free mass, B_FFM and layer thickness of the fat free mass, FFM/H for different levels of muscle tone.

Fig. 13 illustrates an example of a relation between an adipose mass perfusion coefficient, B_AM, and the layer thickness of adipose mass, AM/H.

Fig. 14 shows a semi-quantitative effect on B_FFM and the reducing function, AM_B_FFM_Red, depending on different degrees of muscle tone of an individual.

Fig. 15 shows examples of relations between a reducing function AM_B_FFM_Red and the layer thickness in adipose mass, AM/H for different levels of muscle tone.

## DETAILED DESCRIPTION OF EMBODIMENTS

Known solutions to determine hemodynamic parameters of a subject show quite different percentage errors, e.g. for the measurement of cardiac output (CO), which is an important differentiator between the various CO measurements. Furthermore, the performance of known solutions varies over patient populations. Therefore, an approach that provides more accurate measurements of hemodynamic parameters such as stroke volume (SV) and CO and that performs steadily over various patient populations is desired. Personalization of the measurement to the patient conditions is one of the elements provided according to the present invention to provide a solution.

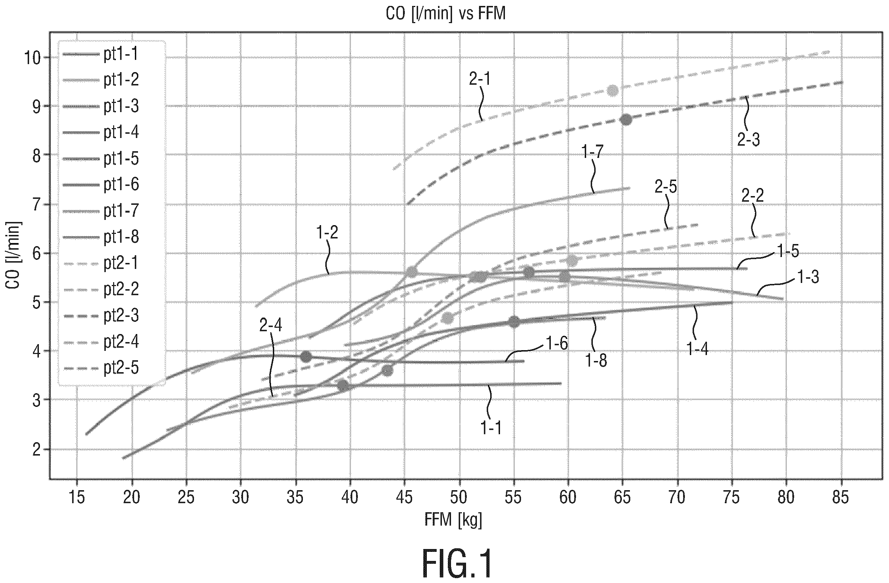

Currently, hemodynamic measurements can make use of a regression model that relates fat-free mass (FFM) to CO. Fig. 1 shows a diagram with examples of a relationship between CO and FFM for several subjects (eight patients (pt1-1 - pt1-8) and five healthy volunteers (pt2-1 - pt2-5)) as used by an exemplary HDM algorithm. For every patient/subject, a different curve is determined by the exemplary algorithm, based on the patient/subject demographics (height, weight, age and gender) and hemodynamic parameters measured by the exemplary algorithm, such as systolic and mean arterial pressure (SAP, MAP) and pulse rate variability (PRvar). The dots on the curves indicate where every individual patient/subject is on his/her curve. Therefore, depending on the specific patient/subject, it can be seen that inaccuracies in FFM can lead to large variations in CO. For instance, inaccuracies in FFM of a few kg will lead to considerable deviations (errors) in the CO estimate for patients pt1-7, pt1-8, pt2-4 and pt2-5. For this small cohort and the exemplary HDM algorithm, an error of 5 kg in FFM can lead to an error of up to 1 liter/min in CO.

Furthermore, FFM is often estimated based on a demographic model using patient height, weight, age and gender, derived e.g. by combining various scientific publications. Such a demographic model is accurate on average for the patient population from which it has been derived. Different models will therefore result for different patient populations.

The application of FFM models can therefore lead to less accurate FFM estimates if the patient does not properly match with the average of the population used to estimate the FFM model. It follows that there is a need to accurately determine FFM for every subject individually so that the desired hemodynamic parameter(s) such as SV and CO can be more accurately determined.

The known SC system and other known systems make use of demographic models to estimate patient characteristics. Such demographic models relate to a general population, leaving room to more specifically tailor the estimates to the individual patients and to more specifically tailor the estimates of computed hemodynamic parameters to the individual patient. The present invention provides a personalized approach that enables a more accurate estimation of FFM and of desired hemodynamic parameters such as SV and CO over various patient populations. Briefly summarized, the present invention presents a device, system and method to personalize/customize the measurement to the individual patient by taking into account specifics of the patient and/or contextual information.

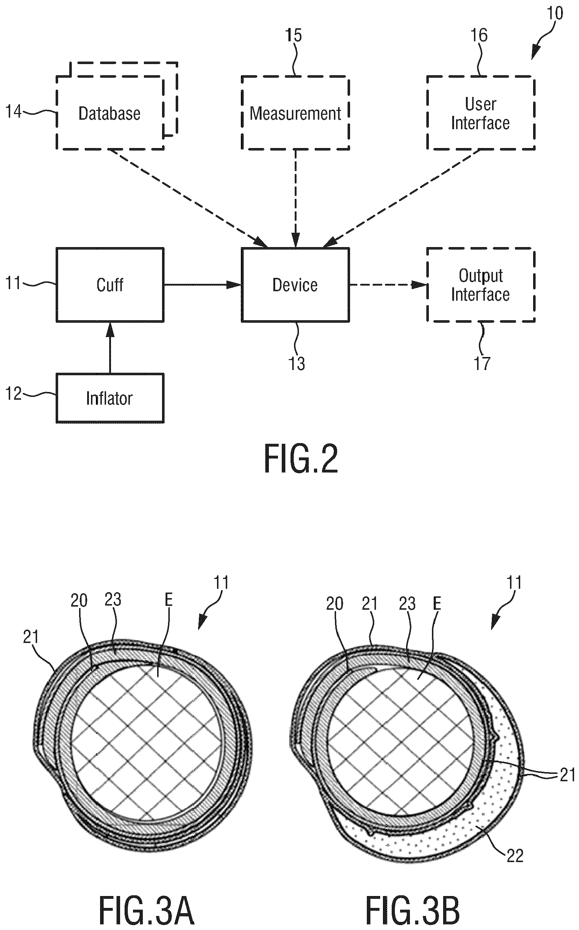

Fig. 2 shows a schematic diagram of an embodiment of a system 10 for determining one or more hemodynamic parameters of a subject according to the present invention. The system 10 comprises an inflatable cuff 11 and an inflator 12 for inflating the inflatable cuff 11, and a device 13 as disclosed herein for determining one or more hemodynamic parameters and as explained below in more detail.

The system 10 may further comprise additional elements such as one or more databases 14 (e.g. an electronic health record, a patient's medical record) storing information, other measurement equipment 15 acquiring information (e.g. body parameters of the subject, such as weight, size, etc., medical images of the patient, etc.), and a user interface 16 for obtaining user input (e.g. patient related data, medication data, etc.). These additional elements 14, 15 and 16 thus provide different pieces of information that can be obtained (i.e. retrieved or received) and used by the device 13.

The system 10 may further comprise an output interface 17 for issuing the determined hemodynamic parameter(s). The output interface 17 may generally be any means that is able to output information in visual or audible form, e.g. in text form, as image or diagram, as sound or spoken words, etc. For instance, the output interface 17 may be a display, a loudspeaker, a touchscreen, a computer monitor, the screen of a smartphone or tablet, etc.

The inflatable cuff 11 may be a Shell Cuff as e.g. disclosed in US 2015/359446 A1, which disclosure is herein incorporated by reference. Fig 3 shows an embodiment of a known inflatable cuff 11, as e.g. disclosed in US 2015/359446 A1, in a deflated state (Fig. 3A) and in an inflated state (Fig. 3B). The cuff 11 (also called Advanced Monitoring Cuff (AMC) or Shell Cuff (SC) herein) uses a kinking-proof shell 20 that is arranged between the pressurization means, comprising a pressure actuator 21 with an air/fluid bag 22, and the body part E (e.g. a subject's upper arm). The kinking-proof shell 20 is arranged (sandwiched) between the pressure actuator 21 with the air/fluid bag 22 and a flexible element 23.

The kinking-proof shell 20 is preferably made from plastic material, such as polyethylene. The thickness of the kinking-proof shell 20 is chosen so that the kinking-proof shell 20 does not buckle when pressure is applied to the air/fluid bag 22, while at the same time the kinking-proof shell 20 is flexible enough to allow for a certain deformation of the kinking-proof shell 20. That is, when pressure is applied to the air/fluid bag 22, overlapping edge regions of the kinking-proof shell 20 move or slide relatively to each other so as to reduce the diameter of the kinking-proof shell 20. However, the kinking-proof shell 20 thereby remains substantially ring-shaped. The kinking-proof shell 20 may comprise individually formed shell elements that are substantially in parallel to each other to better fit or adapt to the shape of the body part E.

The device 13 may be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 13, which may execute a computer program that may be stored in a memory that is accessed by the processor.

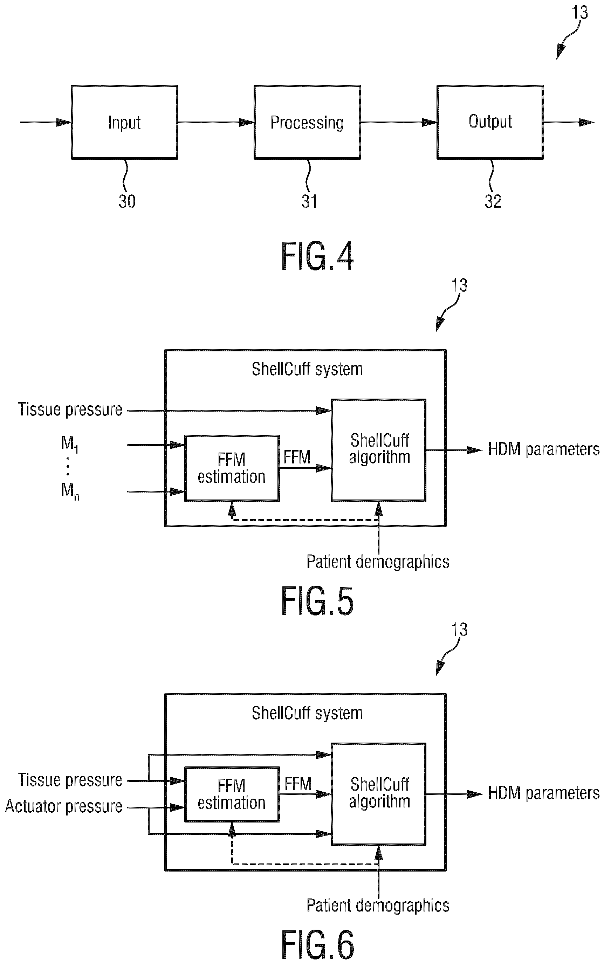

Fig. 4 shows a schematic diagram of a first embodiment of a device 13 for determining one or more hemodynamic parameters according to the present invention. In Fig. 4 the device 13 is illustrated with separate units, which may be units of a processor, computer or circuitry.

The device 13 comprises an input 30 configured to obtain various pieces of information from various sources (e.g. one or more databases 14, other measurement equipment 15 and/or a user interface 16). The input 30 may be directly coupled or connected to the one or more sources or may obtain (i.e. retrieve or receive) information from a storage, buffer, network, or bus, etc. The input 30 may thus e.g. be (wired or wireless) communication interfaces or data interfaces, such as a Bluetooth interface, Wi-Fi interface, LAN interface, HDMI interface, direct cable connect, or a user interface or any other suitable interface allowing signal transfer to the device.

The device 13 further comprises a processing unit 31 configured to process the obtained information to determine one or more hemodynamic parameters. The processing unit 31 may be any kind of means configured to process the information and determine the one or more hemodynamic parameters there from. It may be implemented in software and/or hardware, e.g. as a programmed processor or computer or app on a user device such as a smartphone, smartwatch, tablet, laptop, PC, workstation, etc.

The device 13 further comprises an output 32 configured to output the determined hemodynamic parameter(s). The output 32 may generally be any interface that provides the determined hemodynamic parameter(s), e.g. transmits them to another device or provides them for retrieval by another device (e.g. a smartphone, computer, tablet, etc.). It may thus generally be any (wired or wireless) communication or data interface.

The input 30 obtains the following information: tissue pressure information indicating tissue pressure of tissue of a body portion of the subject, subject demographic information and body-related information related to the subject's body. The body-related information indicates one or more of following:

- one or more parameters of a hemodynamic measurement apparatus used for measuring tissue pressure and/or one or more other measurement parameters,
- one or more subject-related parameters or subject-related information different from the subject demographic information, and

- treatment-related information indicating information about medical treatment of the subject.

The processing unit 31 estimates fat-free mass of the subject from the subject demographic information and the obtained body-related information and determines the one or more hemodynamic parameters of the subject from the obtained tissue pressure information, the estimated fat-free mass and the subject demographic information.

Fig. 5 shows a schematic diagram of a second embodiment of a device 13 according to the present invention. The SC system currently uses tissue pressure (TP) and patient demographics to determine hemodynamic parameters. Based on (additional) measurements (i.e. body-related information) provided to the device 13, indicated by M1 ... Mn, an improved FFM estimate is made which is used in the current SC algorithm to come to better estimates of hemodynamic parameters such as SV and CO.

The measurements used as (additional) inputs to the FFM estimation can be one or more of the following:

a) The currently used actuator pressure and tissue pressure from which an estimate of FFM is obtained. When used for an improved estimate of FFM, tissue pressure and actuator pressure could e.g. be measurements M1 and M2 in the embodiment shown in Fig. 5.

b) One or more of additional measurements / information such as the cuff size, the circumference of the arm / waist / leg of the patient, camera images of the arm and/or the whole body, bioimpedance analysis (BIA) of the patient's thorax and/or body, triceps skinfold thickness (TS) of the arm, near infrared interactance (NIR) of the arm tissue, historical data (medical images) of the patient (such as MRI, CT, X-ray or DXA (dual-energy x-ray absorptiometry) images), which have previously been acquired from the patient, genetic information, administered fluids, urine output, length of stay (LOS) in the hospital or estimated muscle loss during hospital stay. The additional input(s) can be used to obtain an improved estimate of FFM, possibly combined with demographic information as indicated by the dashed arrow Fig. 5. Based on the information a single improved estimate of FFM can be determined or FFM can be dynamically tracked over time.

Fig. 6 shows a schematic diagram of a third embodiment of a device 13 according to the present invention. This embodiment makes use of an estimation of FFM based on the ratio between actuator pressure and tissue pressure. Tissue pressure (TP) and actuator pressure (AP) are used to determine a more accurate FFM estimate tailored to the patient's anatomical characteristics. This embodiment is particularly advantageous for use with a Shell Cuff, because no additional inputs are required so that no additional costs are involved for additional sensors or measurement equipment.

The ratio can e.g. be determined between the (low-pass filtered) tissue and actuator pressures to assess the composition of the patient's arm, based on which FFM is estimated. The ratio can also be used in addition to the patient demographics to determine a more accurate FFM estimate as indicated by the dashed arrow in Fig. 6. As an alternative to the ratio of the (low-pass filtered) pressures, the ratio of the rates at which the actuator and tissue pressure signals increase can be used in a modified embodiment. It is also possible to run actuator pressure sequences and synchronously acquire tissue pressure, where the actuator pressure sequences are tailored to obtain information about the arm anatomy. For instance, the pressure sequences can result in obtaining step responses, harmonic variations. The improved estimate of FFM is an input to the Shell Cuff algorithm which determines HDM parameters such as SV and CO based on tissue pressure and the patient demographics.

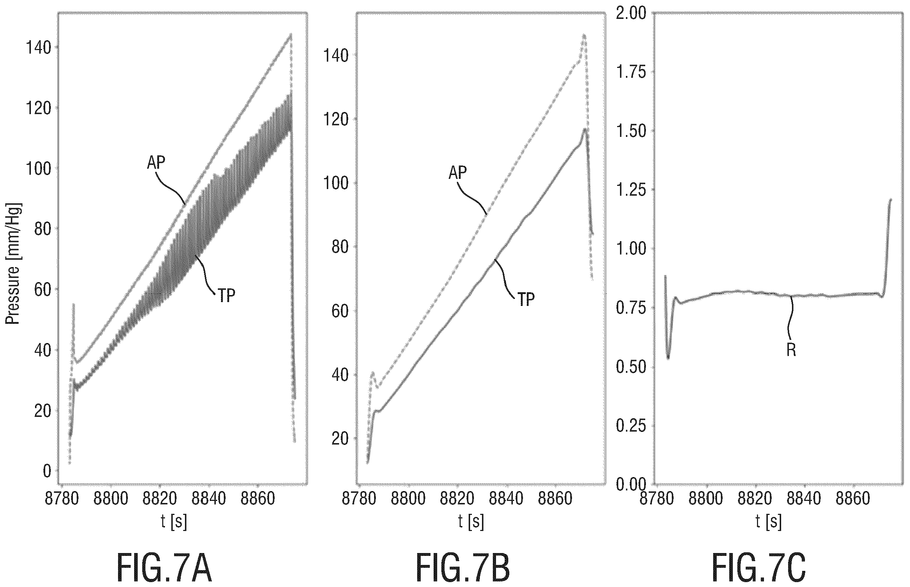

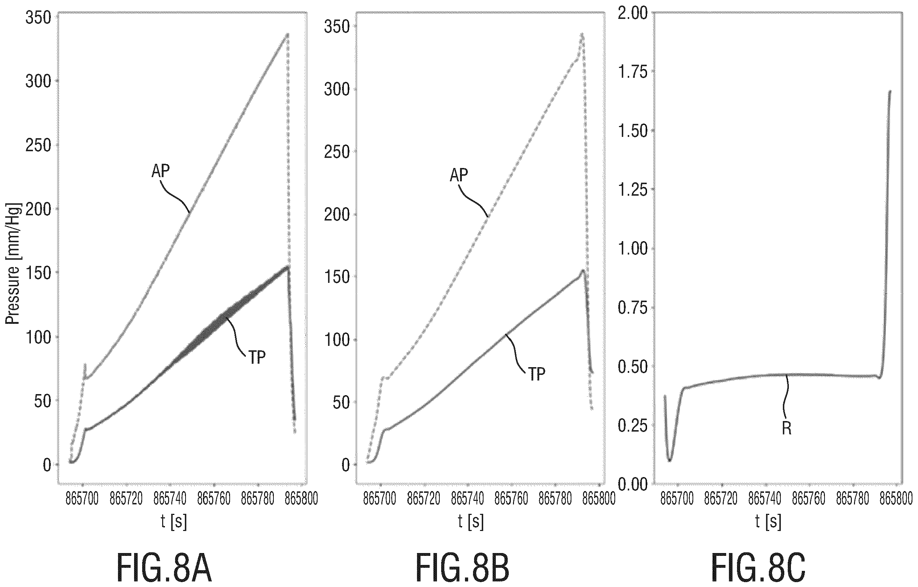

Two examples of the ratio between the low-pass filtered (LPF) actuator pressure (AP) and tissue pressure (TP) signals for different body-mass-index (BMI) are shown in the diagrams depicted in Figs. 7 and 8. This example illustrates that the ratio TP / AP depends on BMI. The ratio is about 0.8 for the person with a BMI of 18, while the ratio is about 0.4 for the person with a BMI of 29. The ratio can therefore provide information about the composition of the arm, which information can be useful to obtain a personalized estimate of FFM.

Fig. 7A shows the raw TP and AP signals, Fig. 7B the low-pass-filtered (LPF) versions of the TP and AP signals and Fig. 7C shows the ratio R of TP (LPF) / AP (LPF) for the person with a BMI of 18. Fig. 8A shows the raw TP and AP signals, Fig. 8B the low-pass-filtered (LPF) versions of the TP and AP signals and Fig. 8C shows the ratio R of TP (LPF) / AP (LPF) for the person with a BMI of 29.

According to another embodiment the estimation of FFM is based on an additional measurement. As shown in Fig. 5, one or more additional measurements can be used by the device 13 to determine the specific/individual patient's FFM, i.e., to personalize the FFM estimate to the patient's characteristics. One or more of the following additional measurements can for instance be used, e.g. in a regression equation to improve the estimate of FFM resulting in a more accurate estimate of FFM:

- Circumference of the arm: this is particularly convenient information as this is measured during the workflow to determine what cuff size to use for the patient. Arm circumference can be used as additional information to the patient demographics to estimate FFM.
- Cuff size: the cuff connector can be made to hold the cuff size information, e.g., in a chip, such that the device can obtain the cuff size information by communication with the connected cuff, as such not requiring any additional manual inputs into the system. Cuff size can also be manually entered, after it has been determined as per the current workflow. Cuff size can be used as additional information to the patient demographics to estimate FFM.

- Circumference of the abdomen / waist: this information can be measured and input into the system via a user interface. Together with the patient demographics information this can lead to an improved estimate of FFM.
- Camera images: camera images of the patient can be input into the device so that it can analyze the body shape in order to improve the estimate of the FFM. An analysis of camera images may allow to assess circumferences of e.g. the arm, leg, waist and/or to assess the "body shape category" to which the patient belongs, e.g., normal/average/regular, underweight, obese, athletic. This can be used as an additional input / additional inputs in a regression equation that assesses fat free mass (FFM) from demographics.
- Bioimpedance analysis (BIA), triceps skinfold thickness or near infrared interactance (NIR): these are additional measurements that can be performed and input to the device in order to improve the estimate of FFM. NIR may also be integrated in the cuff such that the device can directly perform the NIR measurement and obtain the results of the NIR measurement itself.
- Historical patient data: Historical patient data available before the intervention can be used to have a more accurate FFM estimation. An example is an MRI, CT or X-ray scan that the patient had earlier. In obese patients, dual-energy x-ray absorptiometry (DXA) scans may be available. From these scans and medical imaging information the body composition and/or the body fat percentage of the patient can be assessed. The information about the body composition can be introduced by the clinician or nurse in the monitor via a user interface or alternatively this information can be directly obtained by the device via a connection to the patient's electronic medical record (EMR).
- Genetic information: FFM is partially determined by genetic factors. Genotype information of patients can be used as additional information to the patient demographics information to improve estimation of FFM. Specific genetic profiles or specific single-nucleotide polymorphisms (SNPs) can be genotyped from patient's DNA and used as specific markers for personalized FFM estimation. In the future when DNA sequencing is more common this method has the advantage of not requiring additional workflow steps as it is based on evaluating existing patient's genomic data.

The additional information input into the device can be used directly to determine an estimate of FFM or these can be used in conjunction with the patient demographics information to come to an improved estimation of FFM.

NIR (of a patient's arm tissue) is particularly interesting for use with a Shell Cuff, because a NIR device can be integrated into the Shell Cuff having the advantage that no additional workflow steps are required to obtain the NIR measurement.

A regression equation to assess FFM may be determined upfront, e.g. by using the demographic information plus the additional body-related information. A regression equation may be developed based on (a subset of) the possible inputs listed above, against a reference measurement for FFM, where the FFM reference can be obtained from (a combination of) e.g. hydrodensitometry, DXA, isotope dilution water measurements used in a multi-compartment body composition model.

The regression equation can be a generic fitting function, but the shape of the regression equation can also be based on physical principles / insights. For instance, the amount of fat mass is expected to be related to body volume, which for each limb is proportional to cross section times length. Based on this relationship, the square of the circumference measurements may be included instead of the circumference measurements directly as elements of the regression equation. Furthermore, FFM can be considered to be proportional to height squared divided by impedance (as e.g. described in Janmahasatian, S., Duffull, S.B., Ash, S. et al. Quantification of Lean Bodyweight. Clin Pharmacokinet 44, 1051-1065 (2005)). Therefore, the term height squared divided by impedance may be used as one of the elements of the regression equation.

According to another embodiment longitudinal data from the patient may be used for further improvement of the assessment of FFM. The embodiment shown in Fig. 5 can be used to dynamically track the patient's weight and FFM over time. In this implementation, one or more of the following additional inputs may be used: Fluids administered over time (e.g. intravascular fluid administration), urine output, and length of stay (LOS) in a hospital. This information can be obtained from a database, e.g. the electronic medical record (EMR) of the patient. The extra input information/data allows to dynamically track the patient's weight over time in order to assess possible changes in the patient's weight and consequently have a more accurate FFM estimated over time. The information about administered fluids can be used to assess how the patient weight increases over time, while the urine output can be used to assess how the patient weight decreases over time. The length of stay (LOS) in the hospital can be used in a model to estimate loss of muscle tissue as a function of the length of stay in the hospital, possibly related to the specific hospital departments in which the patient has stayed (e.g., on the general ward, ICU). Further, food intake may be considered as additional input to estimate how the patient's weight and FFM dynamically change over time. Here, both the amount and type of food may be used as relevant input in the estimation of the patient weight and FFM. One or more pieces of this additional information may be used as extra input data/information to be added to the model allowing to track the patient weight and FFM over time and consequently obtain more accurate dynamic estimates of FFM.

According to an exemplary implementation the additional body-related information may be used as follows. Actuator pressure and tissue pressure can be used to assess the composition of the arm. At low BMI, the ratio between the TP and AP can be high, e.g. 0.8, and at high BMI the ratio between TP and AP can be low, e.g., 0.4. For instance, at higher BMI more damping of the applied actuator pressure is to be expected. This can be used as an additional input in a regression equation that assesses FFM from demographics. This additional information can result in a more accurate estimate of FFM.

According to another exemplary implementation the additional body-related information may be used as follows. Changes in (the initial estimate of) FFM can be tracked over time by taking into account fluid administration, urine output, food intake and length of stay. The difference between fluid administration and urine output provides information about excess fluid or fluid loss, which leads to higher or lower body weight, respectively. This information can be used in the FFM regression equations to compensate for that. In case of excess fluid, the body weight of the patient can be reduced by the excess amount of fluid in order not to overestimate the FFM. In case of additional fluid loss, the body weight of the patient can be increased by the fluid loss to compensate for the dehydrated state in order to not underestimate the FFM.

Estimates are available that describe a decrease of muscle tissue as a function of length of stay in an ICU (disuse muscle atrophy). The initial estimate of FFM can be reduced by the decrease in muscle tissue as is expected based on the LOS in the ICU.

Food intake may be considered as additional input to estimate how the patient's weight and FFM dynamically change over time. Here, both the amount and type of food may be used in the estimation of the patient weight and FFM. Based on the estimated FFM, the metabolic rate can be estimated, which allows assessment of whether the amount of food intake is expected to increase or decrease the total body weight. If the body weight is expected to increase, this is expected to result in an increase in adipose mass.

A hemodynamic parameter, such as the subject's stroke volume, may be determined by determining a hemodynamic status of the subject from the tissue pressure information waveform and the subject demographic information, and determining a calibrated estimate of the subject's stroke volume by use of the fat-free mass, in particular by adjustment with a perfusion parameter that includes estimates of the fat-free mass and adipose mass of the subject. The calibrated estimate may be determined by determining a first estimate of the subject's stroke volume from the hemodynamic status, and then calibrating the first estimate of the subject's stroke volume into a second estimate of stroke volume by use of the fat-free mass, in particular by adjustment with a perfusion parameter that includes estimates of the fat-free mass and adipose mass of the subject.

In a more detailed embodiment, the subject's stroke volume may be determined by use of a physiology-based prediction model which determines a first dimensionless uncalibrated estimate of stroke volume from the hemodynamic status of the subject as derived from the tissue pressure waveform and the subject's demographic information. The first dimensionless uncalibrated estimate of stroke volume is then calibrated into a second calibrated volumetric estimate of stroke volume by adjustment with a perfusion parameter which includes estimates of the FFM and the Adipose Mass of the subject. Hereby, the FFM may be determined via a regression equation which includes subject demographic information and body-related information, and the Adipose Mass may be determined as the subject's weight minus the estimated FFM. The parameters of the physiology-based prediction model may have been estimated from clinical data.

In the following, the operation of an example of a known SC system shall be explained. Further, it will be explained how this example can be used in the context of the present invention, i.e., how this example may be modified to carry out the present invention.

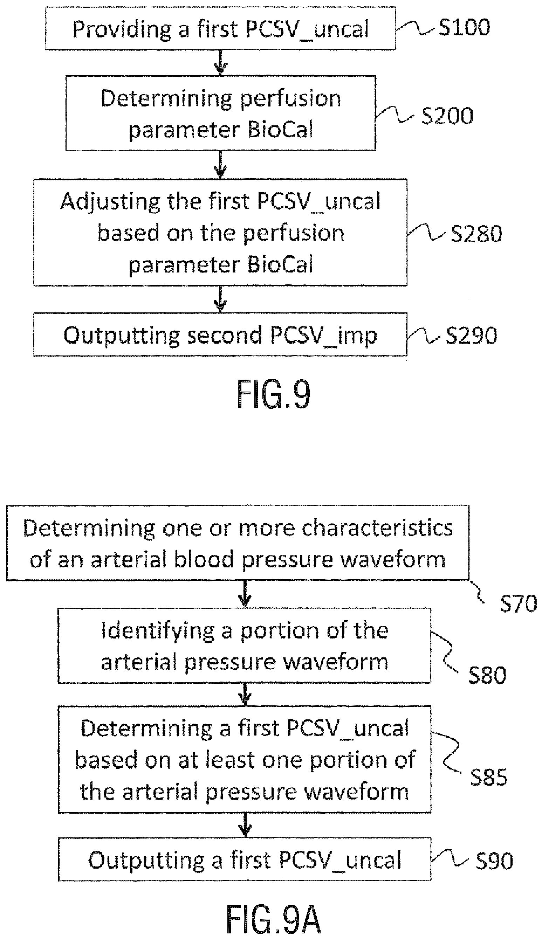

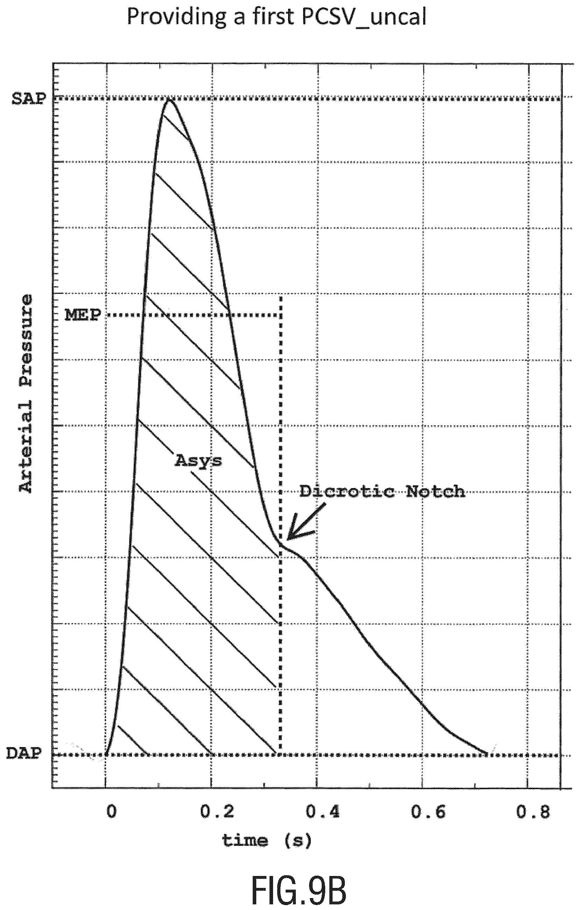

Fig. 9 provides a rough overview of an example of a method used in a known SC system. In this example a biological calibration based on a perfusion parameter, BioCal, of any uncalibrated pulse contour stroke volume, PCSV_uncal, is made by considering a separation of blood flow through fat free mass, FFM and adipose mass, AM.

According to an embodiment of the present invention, AM may be used as additional input, determined as total body weight minus the improved estimate of FFM (see also equation (3) below). The total body weight may be obtained from the subject demographic information and an improved estimate of FFM may be obtained as described above. It may be advantageous to estimate both, FFM and AM, individually because the perfusion of these types of tissues is different.

According to the flow chart shown in Fig. 9 in step S 100, the uncalibrated pulse contour stroke volume, PCSV_uncal, also called first PCSV, is provided. In a next step S200 the perfusion parameter, BioCal, is determined. This will be described in further detail below. After having determined the perfusion parameter, BioCal, this perfusion parameter, BioCal, is applied to the first PCSV_uncal in step S280 to provide the second PCSV_imp in step S290.

Fig. 9A provides an exemplary flow chart for providing the first PCSV_uncal. In step S70 a determination or measurement of one or more characteristics of an arterial blood pressure waveform is made. This can be made invasively or noninvasively. So, the result of such determination and/or measurements are parameters of a blood pressure curve of an individual which allow to identify, in step S80, a portion of such blood pressure curve. So, values like amplitude, area enclosed by the curve, et cetera, can be considered to determine the first PCSV_uncal in step S85 and to output the same in step S90. The methods for providing a PCSV_uncal are known in this technological field. A known example for providing such PCSV_uncal is the Wesseling Algorithm (Wesseling algorithm in Chen et al. Comput Cardiol. 2009 Jan 1. The Effect of Signal Quality on Six Cardiac Output Estimator). However, also other algorithms can be used to provide the PCSV_uncal.

An example for providing such PCSV_uncal is illustrated in Fig. 9B. Fig. 9B illustrates a portion of a blood pressure curve measured, e.g. by use of a high-fidelity oscillometry hydraulic blood pressure cuff or invasively by direct blood pressure measurement using a fluid column and a pressure transducer. In a simple exemplary way the first PCSV_uncal is determined by using blood pressure, pulse pressure, the systolic pressure area, Asys, indicated hatched, and other parameters such as aortic compliance and arterial impedance. The systolic pressure area, Asys, is the area enclosed by the arterial pressure curve during the systole (i.e. ejection phase of the left ventricle), which is defined from the time of the start of the systole, peaking with a systolic arterial blood pressure, SAP, to the beginning of the dicrotic notch, which corresponds to the closure of the aortic valve. Its baseline is defined either as horizontal line at the level of diastolic arterial blood pressure, DAP, of the preceding pulse (as in Fig. 9B), or as straight line between the DAP of the preceding pulse and the DAP of the pulse under consideration.

The mean left ventricular ejection pressure, MEP, is the mean value of the arterial pressure curve during the systole. The MEP is illustrated in Fig. 9B as a horizontal line.

As indicated above, the stroke volume of an individual depends on individual biometric/demographic parameters like body weight, W, comprising of fat free mass, FFM, and adipose mass, AM, body height, H, age and gender.

So the first major determinant of a stroke volume, SV, in an algorithm using an aggregate formula for determining the SV, are the individual biometric/demographic parameters.

The second major determinant of a SV of an individual is the hemodynamic status. So, a pulse contour evaluation of a measured arterial blood pressure pulse waveform results in dynamic input parameters from which an uncalibrated PCSV (PCSV_uncal) is calculated.

In an example BioCal provides a calibration function for scaling any given PCSV_uncal to the absolute PCSV value according to the individual biometric/demographic input parameters.

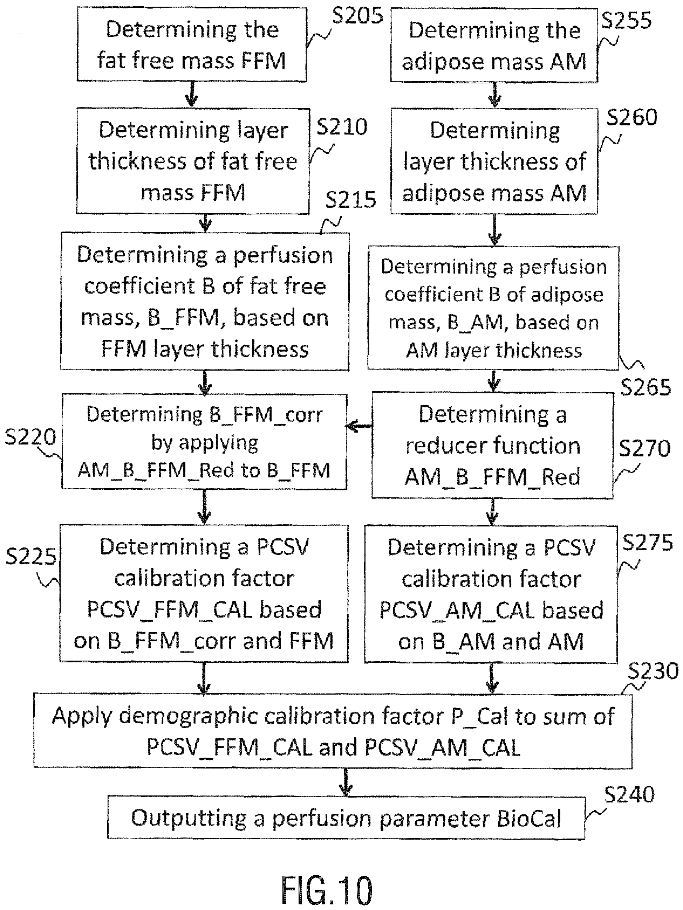

In Fig. 10 a detailed flow chart for determining the perfusion parameter, BioCal, is given. As indicated in this example a separation of fat free mass and adipose mass is made. Thus, in step S205 and S255, the fat free mass, FFM and the adipose mass, AM are determined respectively.

The fat free mass, FFM, and adipose mass, AM, determination is explained in detail in the following.

FFM (kg) and AM (kg) represent the two portions of an individual's body weight, W (kg), according to formula 1

$$W \ (kg) = FFM \ (kg) + AM \ (kg)$$

FFM is the metabolically highly active mass of the body with large oxygen consumption and therefore accounts for the, by far, largest portion of SV. AM is the mass of the metabolically very little active tissue with low oxygen consumption and therefore accounts for a, by far, minor portion of SV.

For estimation of FFM (kg) and AM (kg) the equations of Yu, Heitmann and Janmahasatian in Yu et al. BMC Pharmacol Toxicol. 2013 Oct 14. Lean body mass: the development and validation of prediction equations in healthy adults are used according to this example and are averaged to:

$$FFM \ (kg) = (\ 56.128 \ kg + (1.3016 + 9270 \ / \ (8780 + 244 \ m^2/kg \ \bullet BMI \ (kg/m^2))) \bullet W \ (kg) - 2.2268 \ m^2 \bullet BMI \ (kg/m^2) - 0.1134 \ kg/y \bullet age \ (y) \ ) \ / \ 3,$$

for female individuals

$$FFM \ (kg) = (\ 66.068 \ kg + (1.4138 + 9270 \ / \ (6680 + 216 \ m^2/kg \ \bullet BMI \ (kg/m^2))) \bullet W \ (kg) - 2.2268 \ m^2 \bullet BMI \ (kg/m^2) - 0.1134 \ kg/y \bullet age \ (y) \ ) \ / \ 3,$$

for female individuals

$$AM \ (kg) = W \ (kg) - FFM \ (kg)$$

Equation (2) is an example for prediction of FFM and can be replaced by further improved prediction equations. According to the present invention, FFM prediction equations as known are no longer used as such and as described above, but body-related information is additionally included to achieve a personalized and therefore more accurate estimate of FFM. This then also gives a more accurate estimate of AM via AM = body weight - FFM as given by equation (3), which may still be used. For instancce, equation (2) may be modified into

$$(2') \qquad FFM(kg) = a_0 + a_1 \cdot W + a_2 \cdot H + a_3 \cdot BMI + a_4 \cdot age + a_5 \cdot gender + a_6 \cdot dTP/dAP + a_7 \cdot$$

$$cuffsize + a_8 \cdot ArmCircumference + a_9 \cdot LegCircumference + a_{10} \cdot WaistCircumference + a_{11} \cdot$$

$$TricepsSkinfoldThickness + a_{12} \cdot NIR + a_{13} \cdot BodyType + a_{14} \cdot GeneticInformation + a_{15} \cdot Z + a_{16} \cdot$$

$$(AdministeredFluids - UrineOutput) + a_{17} \cdot LengthOfStay + a_{18} \cdot CaloryIntake + a_{19} \cdot Medication,$$

with regression coefficients $a_i$, which may be positive, negative or zero, dTP the increase in tissue pressure during an inflation of the cuff or the rate at which the tissue pressure increased during an inflation of the cuff, dAP the increase in actuator pressure during an inflation of the cuff or the rate at which the actuator pressure increased during an inflation of the cuff, body type the "body shape category" to which the patient belongs as determined via e.g. analysis of camera images, Z the impedance as measured via bioimpedance analysis (BIA), (AdministeredFluids - UrineOutput) describing the hydration state of the patient, LengthOfStay the length of stay in the hospital and CaloryIntake the amount of calories consumed via food intake.

Alternatively, other than linear relationships between FFM and specific body-related parameters may be taken into account if this can be expected from e.g. physical models, e.g., via

$$(2'') \qquad FFM(kg) = b_0 + b_1 \cdot W + b_2 \cdot H + b_3 \cdot BMI + b_4 \cdot age + b_5 \cdot gender + b_6 \cdot dTP/dAP + b_7 \cdot$$

$$cuffsize + b_8 \cdot ArmCircumference^2 + b_9 \cdot LegCircumference^2 + b_{10} \cdot WaistCircumference^2 + b_{11} \cdot$$

$$TricepsSkinfoldThickness + b_{12} \cdot NIR + b_{13} \cdot BodyType + b_{14} \cdot GeneticInformation + b_{15} \cdot (H^2/Z) + b_{16} \cdot$$

$$(AdministeredFluids - UrineOutput) + b_{17} \cdot LengthOfStay + b_{18} \cdot CaloryIntake + b_{19} \cdot Medication,$$

with regression coefficients $b_i$, which may be positive, negative or zero, where it has been used that FFM is expected to depend on the square of the circumference measurements and to be related to the impedance via $H^2/Z$. I.e., prior knowledge about the mathematical relationship with specific body-related parameters can be included in the FFM model.

Alternatively, the fluid-related body-related parameters can each be assigned an individual regression coefficient, instead of using their difference in the regression equations, and regression coefficients can also be made dependent on other parameters, as e.g.

$$(2''') \qquad FFM(kg) = c_0 + c_1 \cdot W + c_2 \cdot H + c_3 \cdot BMI + c_4 \cdot age + c_5 \cdot gender + c_6 \cdot dTP/dAP + c_7 \cdot$$

$$cuffsize + c_8 \cdot ArmCircumference^2 + c_9 \cdot LegCircumference^2 + c_{10} \cdot WaistCircumference^2 + c_{11} \cdot$$

$$TricepsSkinfoldThickness + c_{12} \cdot NIR + c_{13} \cdot BodyType + c_{14} \cdot GeneticInformation + c_{15} \cdot (H^2/Z) + c_{16} \cdot$$

$$AdministeredFluids + c_{17} \cdot UrineOutput + c_{18}(DepartmentType) \cdot LengthOfStay + c_{18}(W, FFM_{previous}) \cdot$$

$$CaloryIntake + c_{19} \cdot Medication,$$

with regression coefficients $c_i$, which may be positive, negative or zero, where the regression coefficient of the LengthOfStay depends on the hospital department where the patient stayed, such as the Intensive Care Unit (ICU), Medium Care Unit (MCU) or General Ward (GW), and where the regression coefficient of the CaloryIntake depends on the current weight of the patient and the previously measured FFM.

Alternatively, gender may not be directly used as a term in the regression equations, but instead different sets of regression coefficients may be determined for each gender. The value of the regression coefficients may be dependent on the gender, i.e., the coefficient values may be a function of gender.

The regression coefficients of the equations above may be determined upfront, e.g. by using the demographic information plus the additional body-related information and determining a regression against a reference measurement for FFM, where the FFM reference can be obtained from (a combination of) e.g. hydrodensitometry, DXA, isotope dilution water measurements used in a multi-compartment body composition model.

It shall be noted that equation (2) and its variations explained above are just example model equations of linear models. It is also possible to extend them e.g. by interaction terms, such as with x*y or quadratic terms. For adolescents, children and infants other appropriate FFM and AM estimation equations as e.g. published in literature may be used and averaged. FFM estimation equations have for instance specifically been developed for adolescents in Ripka WL, Orsso CE, Haqq AM, Prado CM, Ulbricht L, Leite N. Validity and accuracy of body fat prediction equations using anthropometries measurements in adolescents. Eat Weight Disord. 2021 Apr;26(3):879-886.

It is important to note that such FFM and AM estimations relate to the corresponding condition in a healthy status and in general assume that 1) the water contents of FFM is constant and 2) AM contains no or very little water. Hence, when applied in a clinical condition of a patient, not the actual weight, which may be falsified by e.g. generalized edema or an effusion, may be entered in such equations but only the last known pre-illness weight.

Based on the determination of the FFM and AM a relationship between stroke volume, SV(ml) and the FFM and AM can be given

$$SV(ml) \sim B\_FFM \ (ml/kg) \cdot FFM \ (kg) + B\_AM \ (ml/kg) \cdot AM \ (kg) + \ldots + B\_Const \ (ml),$$

where B_FFM is a perfusion coefficient related to FFM perfusion, B_AM is a perfusion coefficient related to AM perfusion and B_Const accounts for other influences on SV. The perfusion coefficients B_FFM and B_AM are determined in Steps S215 and S265 respectively.

In Collis et al. Circulation. 2001 Feb 13;103(6). Relations of stroke volume and cardiac output to body composition: the strong heart study and Corden et al. J Cardiovasc Magn Reson. 2016 May 31;18(1):32. Relationship between body composition and left ventricular geometry using three dimensional cardiovascular magnetic resonance, it has been found to differentiate B_FFM and B_AM for women and men.

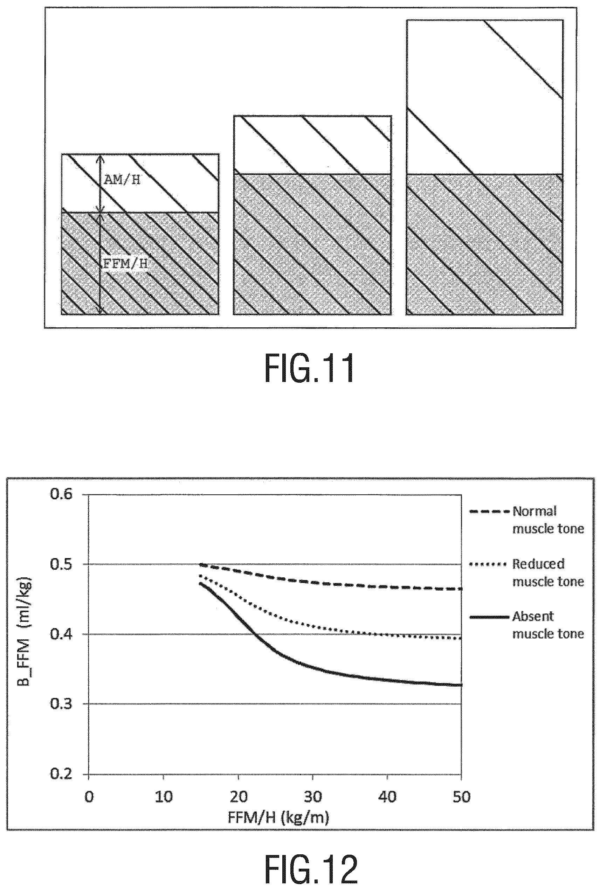

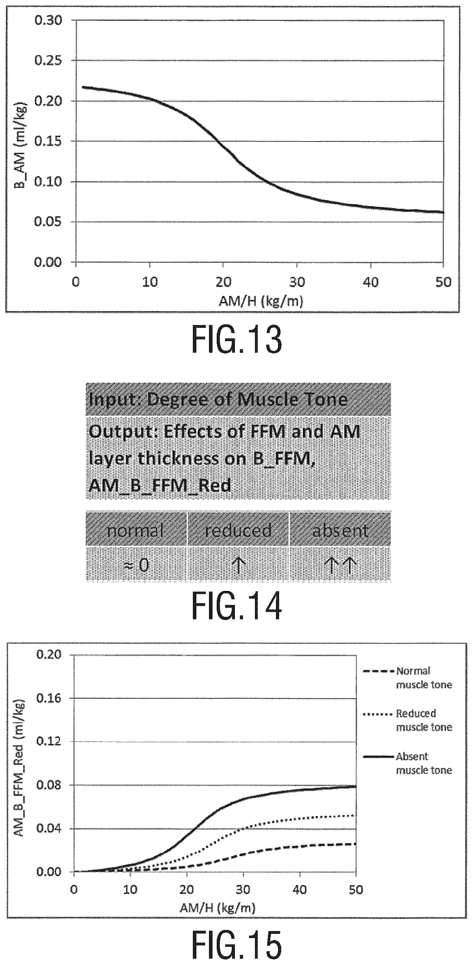

Applying different, but constant perfusion coefficients B_FFM and B_AM considers a difference in the FFM's blood flow per mass FFM_perf (ml/min/0.1 kg) and in the AM's blood flow per mass AM_perf (ml/min/0.1 kg) but assumes a perfusion behavior of the FFM and AM portion independent from the relationship of FFM and AM. However, B_FFM and B_AM are not constant (Fig. 12 and Fig. 13).

The blood flow through AM, AM_perf (ml/min/0. 1kg), referred to AM (kg) and the perfusion coefficient B_AM strongly depend on the body mass' AM share AM% = AM (kg)/W (kg), indicating that perfusion coefficient B_AM has to be adapted individually. However, the perfusion coefficient B_AM dependency on AM% is not sufficient to describe the variability of B_AM, because it includes no information of the absolute level of AM.

Thus, according to the example a simple model is provided that uses AM and FFM distributions over height of an individual to determine the influences on perfusion coefficients B_AM and B_FFM.

In a simplifying model AM and FFM are represented as two corresponding layers as illustrated in Fig. 11. The FFM layer thickness is estimated by FFM/H in step S210. The AM layer thickness is estimated by AM/H, in step S260.

Normal values of FFM/H, AM/H and other biometric/demographic parameters are given in the table below.

| Normal values of biometric/demographic parameters | | | | | | | | |
|---|---|---|---|---|---|---|---|---|
| Age (y) | Gender | Height (m) | Weight (kg) | BMI (kg/m²) | FFM (kg) | AM (kg) | FFM/H (kg/m) | AM/H (kg/m) |
| 50 | female | 1.70 | 60 | 21 | 41 | 19 | 24.0 | 11.3 |
| 50 | male | 1.80 | 70 | 22 | 56 | 14 | 31.2 | 7.7 |

The model shown in Fig. 11 is based on these considerations and appropriate perfusion functions have been developed to describe the behavior of perfusion coefficients B_FFM and B_AM.

In Fig. 11 the white portion is the adipose mass, AM, and the gray portion is the fat free mass, FFM. As shown in Fig. 11 the AM (white) and FFM (gray) perfusion depends on layer thicknesses AM/H and FFM/H, where H = body height. The density of hatching indicates the level of perfusion - dense hatching = higher perfusion (left side in Fig. 11), wide hatching = lower perfusion (right side in Fig. 11). As can be easily recognized in case of a thin FFM layer the perfusion through FFM is higher as in case of a thicker FFM layer. But also the perfusion through AM and FFM in case of a thinner AM layer is higher than in case of a thicker AM layer.

In patients with low or without muscular tone, e.g. unconscious or anaesthetized and neuro muscular blockage patients, the muscles lack their structural supporting function. Therefore, the weight of FFM layers influences perfusion coefficient B_FFM gravitationally, because this weight compresses large parts of FFM, i.e. the muscles, inner abdominal organs etc. Thus, the perfusion coefficient B_FFM decreases with increasing layer thickness of FFM/H.

Also it has been recognized that perfusion coefficient B_FFM is reduced with increasing layer thickness AM/H of the adipose mass, AM.

In patients without muscular tone an AM layer thickness reduces the perfusion coefficient B_FFM gravitationally with increasing AM layer, because the increasing subcutaneous fat layer additionally compresses large parts of FFM.

Furthermore, it has been found that the perfusion coefficient B_AM decreases gravitationally with increasing AM/H due to lack of a supporting structure in adipose tissue. Moreover, the perfusion coefficient B_AM is not influenced by the layer thickness of FFM/H.

So, a function B_FFM(FFM/H) as shown in Fig. 12 decreases with increasing FFM/H and preferably is a nonlinear function, e.g. an arctan, sigmoid or logarithmic function, preferably described by

$$\text{B\_FFM(FFM/H) (ml/kg)} = \text{a\_FFM (ml/kg)} \bullet \arctan[(\text{FFM/H (kg/m)} - \text{b\_FFM (kg/m)}) \bullet \text{c\_FFM (m/kg)}] + \text{d\_FFM (ml/kg)},$$

wherein a_FFM, b_FFM, c_FFM and d_FFM are individual FFM related calibration coefficients and constants with B_FFM being in a range of 0.2 ... 0.7 ml/kg.

Exemplary courses of B_FFM(FFM/H) are given in Fig. 12 showing relations between B_FFM and FFM/H for different levels of muscle tone showing nonlinearly decreasing B_FFM with increasing FFM/H.

According to the flow chart in Fig. 10 the layer thickness of FFM and AM needs to be determined in step S210 and S260, respectively. This is made using the model given in Fig. 11. Based on these layer thicknesses FFM/H and AM/H the respective perfusion coefficients B_FFM and B_AM are determined in step S215 and S265, respectively.

In a next step S220 the perfusion coefficient B_FFM is adjusted by applying a reducing function, AM_B_FFM_Red(AM/H). The reducing function may also be replaced by an attenuation function. In the following only the application of a reducing function is described. The reducing function, AM_B_FFM_Red describes the effect of the thickness of AM on the perfusion coefficient B_FFM. The reducing function, AM_B_FFM_Red increases with increasing AM/H and preferably is a nonlinear function, e.g. an arctan, sigmoid or logarithmic function, exemplary shown in Fig. 15 for different levels of muscle tone.

The reducing function, AM_B_FFM_Red, increases with increasing AM/H and preferably is a nonlinear function, e.g. an arctan, sigmoid or logarithmic function, described in formula 6,

$$\text{AM\_B\_FFM\_Red(AM/H) (ml/kg)} = \text{a\_att (ml/kg)} \bullet \arctan[(\text{AM/H (kg/m)} - \text{b\_att (kg/m)}) \bullet \text{c\_att (m/kg)}] + \text{d\_att (ml/kg)},$$

where a_att, b_att, c_att and d_att are individual AM related calibration coefficients and constants with AM_B_FFM_Red being in a range of 0 ... 0.2 ml/kg.

Going back to step S220 in Fig. 10, a corrected perfusion coefficient, B_FFM_corr, is the difference of the preliminary B_FFM(FFM/H) and AM_B_FFM_Red(AM/H), described in formula 7

$$\text{B\_FFM\_corr (FFM/H, AM/H) (ml/kg)} = \text{B\_FFM (FFM/H) (ml/kg)} - \text{AM\_B\_FFM\_Red (AM/H) (ml/kg)}$$

So, it has been found that depending on the level of consciousness, anesthesia and/or muscle relaxation the perfusion coefficient B_FFM(FFM/H) and the reducing function AM_B_FFM_Red(AM/H) have to be adapted.

Fig. 14 illustrates a semi-quantitative effect on B_FFM and on the reducing function, AM_B_FFM_Red, which depends on different degrees of muscle tone of an individual. In the state of absent muscle tone (e.g. fully anaesthetized and muscle-relaxed patients) the effects of FFM and AM layer thickness on B_FFM and AM_B_FFM_Red are most pronounced as shown in Figs. 12 and 15. In the state of normal, resting muscle tone the aforementioned effects are least pronounced, and the state of reduced muscle tone (e.g. unconscious patients) the effects are moderately pronounced.

But, also the function B_AM(AM/H) decreases with increasing AM/H and preferably is a nonlinear function, e.g. an arctan, sigmoid or logarithmic function. It is given by formula 8,

$$(8) \qquad B\_AM(AM/H) \ (ml/kg) = a\_AM \ (ml/kg) \bullet \arctan[(AM/H \ (kg/m) - b\_AM \ (kg/m)) \bullet$$

$$c\_AM \ (m/kg)] + d\_AM \ (ml/kg).$$

where a_AM, b_AM, c_AM and d_AM are individual AM related calibration coefficients and constants with B_AM being in a range of 0 ... 0.4 ml/kg.

An exemplary course of B_AM(AM/H) is given in Fig. 13 showing that the perfusion coefficient B_AM of the adipose mass AM decreases with increasing layer thickness AM/H of the AM.

Alternatively, perfusion coefficient B_AM(AM/H) can be replaced by a function with similar behavior describing the AM perfusion decreasing with increasing AM/H. The perfusion coefficient B_FFM(FFM/H) can be replaced by a function with similar behavior describing the FFM perfusion decreasing with increasing FFM/H and further decreasing with increasing AM/H.

Further alternatively, AM/H is replaced by the ratio of AM and body surface area AM/BSA or by $AM/H^2$ and FFM/H is replaced by the ratio of FFM and body surface area FFM/BSA or by $FFM/H^2$.

Going back to Fig. 10, in steps S225 and S275 individual static pulse contour stroke volume, PCSV calibration factors related to fat free mass FFM and adipose mass AM are determined by use of the perfusion coefficients B_FFM_corr and B_AM, respectively.

$$(9) \qquad PCSV\_FFM\_Cal \ (ml) = B\_FFM\_corr \ (ml/kg) \bullet FFM \ (kg),$$

$$(10) \qquad PCSV\_AM\_Cal \ (ml) = B\_AM \ (ml/kg) \bullet AM \ (kg)$$

To obtain the second PCSV_imp of an individual, the first PCSV_uncal is calibrated by using the individual static pulse contour stroke volume, PCSV calibration factors PCSV_FFM_Cal, PCSV_AM_Cal.

$$(11) \qquad PCSV \ (ml) = PCSV\_uncal \bullet (PCSV\_FFM\_Cal \ (ml) + PCSV\_AM\_Cal \ (ml) \ ) \bullet P\_Cal$$

wherein PCSV_uncal and P_Cal have no dimension.

Furthermore, an additional static, demographic calibration factor P_Cal is applied, as shown in step S230 in Fig. 10.

The first PCSV_uncal reflects the actual hemodynamic state of the individual, depending on dynamic influences such as cardiac preload (i.e. dependent on intrathoracic blood volume) and performance (i.e. contractility), aortic compliance, arterial impedance and blood pressure (i.e. cardiac afterload).

P_Cal is a calibration factor that depends on the individual's biometric/demographic characteristics additionally to FFM and AM, that influence the level of SV.

The static, biometric/demographic calibration factor P_Cal can be a sum or product of various biometric/demographic parameters, e.g. as shown in formula 12,

$$(12) \qquad P\_Cal = H \ (m) \bullet coeff\_H \ (1/m) + age \ (yrs) \bullet coeff\_age \ (1/yrs) + const\_gender$$

with coeff_H referring to the individual's body height, coeff_age referring to the individual's age (compliance of the arterial system), const_gender has no dimension and is referring to the individual's biological gender.

In case of transgender a semi-quantitative input information about the individual's physique is needed. This assessment can e.g. be a value within the range 0.0 to 1.0, e.g. 0.7, with male $\triangleq$ 1.0 and female $\triangleq$ 0.0

The static, biometric/demographic calibration factor P_Cal can be refined by an additional constant that has no dimension referring to additional parameters influencing the PCSV level that have not been considered by the input data H, age and gender:

$$(13) \qquad P\_Cal = H\,(m) \cdot coeff\_H\,(1/m) + age\,(yrs) \cdot coeff\_age\,(1/yrs) + const\_gender +$$

$$const\_BC$$

The PCSV calculation can also be further refined by an additional correction constant, const_BC, referring to additional parameters influencing PCSV that have not been considered in the above described PCSV calibration procedure.

So, based on the PCSV_FFM_Cal and PCSV_AM_Cal and demographic calibration factor P_Cal the perfusion parameter BioCal is determined in step S240, which is applied to the first PCSV_uncal.

The second PCSV_imp then adds up to:

$$(14) \qquad PCSV\_imp\,(ml) = PCSV\_uncal \cdot [(PCSV\_FFM\_Cal\,(ml) + PCSV\_AM\_Cal\,(ml) +$$

$$const\_corr\,(ml)) \cdot P\_Cal]$$

$$BioCal\,(ml) = (PCSV\_FFM\_Cal\,(ml) + PCSV\_AM\_Cal\,(ml) + const\_corr\,(ml)) \cdot P\_Cal$$

$$PCSV\_imp\,(ml) = PCSV\_uncal \cdot BioCal\,(ml)$$

The coefficients and constants of the functions of static input parameters yielding the calibrating constants PCSV_FFM_Cal, PCSV_AM_Cal and P_Cal are determined in an evaluation population.

According to the example described above, BioCal is the internal parameter used to calibrate the uncalibrated SV. The uncalibrated SV is dimensionless, and by multiplying the uncalibrated SV by perfusion parameter BioCal, the calibrated SV in ml is obtained, which may then be output. In Equation (14), PCSV_imp may be the output to the clinician.

In practice, even further regression equations may be applied to PCSV_imp prior to outputting the value, but in essence PCSV_imp would be the SV that would be output to the clinician. Multiplication of PCSV_imp by pulse rate PR would then give the CO which may be output to the clinician. The hemodynamic parameters that may be determined and output according to the present invention may e.g. be PCSV_imp and CO.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining one or more hemodynamic parameters of a subject, the device comprising circuitry configured to:

- obtain tissue pressure information indicating tissue pressure of tissue of a body portion of the subject;
- obtain subject demographic information;
- obtain body-related information related to the subject's body indicating one or more of
- one or more parameters of a hemodynamic measurement apparatus used for measuring tissue pressure and/or one or more other measurement parameters,
- one or more subject-related parameters or subject-related information different from the subject demographic information, and

- treatment-related information indicating information about medical treatment of the subject;
- estimate fat-free mass of the subject from the subject demographic information and the obtained body-related information; and
- determine the one or more hemodynamic parameters of the subject from the obtained tissue pressure information, the estimated fat-free mass and the subject demographic information.

2. Device as claimed in claim 1,
   wherein the circuitry is configured to use, as parameters of a hemodynamic measurement apparatus used for measuring tissue pressure or as other measurement parameters, one or more of actuator pressure and cuff size.

3. Device as claimed in any one of the preceding claims,
   wherein the circuitry is configured to use, as subject-related parameters or subject-related information, one or more of circumference of the subject's arm, circumference of the subject's waist or abdomen, circumference of the subject's leg, one or more images of the subject's arm, one or more images of the subject's body, triceps skinfold thickness of the subject's arm, near infrared interactance of the subject's arm tissue, one or more medical images of the subject, and genetic information of the subject, urine output of the subject, and amount and/or type of food consumed by the subject.

4. Device as claimed in any one of the preceding claims,
   wherein the circuitry is configured to use, as treatment-related information, one or more of a bioimpedance analysis of the subject's thorax and/or body, administered fluids or medication, length of stay in a hospital, and estimated muscle loss during hospital stay.

5. Device as claimed in any one of the preceding claims,
   wherein the circuitry is configured to use, as subject demographic information, one or more of height, weight, age and gender of the subject.

6. Device as claimed in any one of the preceding claims,
   wherein the circuitry is configured to

   - use, as body-related information, an actuator pressure of an actuator used for inflating a cuff of the hemodynamic measurement apparatus;
   - determine a ratio between the actuator pressure and the tissue pressure or between the rates at which the actuator pressure and the tissue pressure increase during inflation of the cuff; and
   - use the determined ratio in the step of estimating the fat-free mass and/or the step of determining the one or more hemodynamic parameters.

7. Device as claimed in claim 6,
   wherein the circuitry is configured to provide an actuator control signal to the actuator to apply actuator pressure sequences and to obtain actuator pressure values and tissue pressure values while the actuator sequences are applied.

8. Device as claimed in any one of the preceding claims,
   wherein the circuitry is configured to track the subject's weight over time based on one or more of the subject-related parameters, the subject-related information and the treatment-related information and to update the estimation of the fat-free mass in case of a weight change.

9. Device as claimed in any one of the preceding claims,
   wherein the circuitry is configured to estimate fat-free mass by use of a regression equation.

10. Device as claimed in any one of the preceding claims,
    wherein the circuitry is configured to determine the one or more hemodynamic parameters by

    - determining a hemodynamic status of the subject from the tissue pressure information waveform and the subject demographic information, and
    - determining a calibrated estimate of the hemodynamic parameter, in particular the subject's stroke volume, by use of the fat-free mass, in particular by adjustment with a perfusion parameter that includes estimates of the fat-free mass and adipose mass of the subject.

**11.** Device as claimed in claim 10,
wherein the circuitry is configured to

- determine a first estimate of a hemodynamic parameter, in particular the subject's stroke volume, from the hemodynamic status, and
- calibrate the first estimate of the hemodynamic parameter, in particular the subject's stroke volume, into a second estimate of stroke volume by use of the fat-free mass, in particular by adjustment with a perfusion parameter that includes estimates of the fat-free mass and adipose mass of the subject.

**12.** System for determining one or more hemodynamic parameters of a subject, the system comprising:

- an inflatable cuff;
- an inflator configured to inflate the inflatable cuff; and
- a device as claimed in any one of the preceding claims.

**13.** System as claimed in claim 12,
wherein the cuff is a shell cuff.

**14.** Method for determining one or more hemodynamic parameters of a subject, the method comprising:

- obtaining tissue pressure information indicating tissue pressure of tissue of a body portion of the subject;
- obtaining subject demographic information;
- obtaining body-related information related to the subject's body indicating one or more of
- one or more parameters of a hemodynamic measurement apparatus used for measuring tissue pressure and/or one or more other measurement parameters,
- one or more subject-related parameters or subject-related information different from the subject demographic information, and
- treatment-related information indicating information about medical treatment of the subject;
- estimating fat-free mass of the subject from the subject demographic information and the obtained body-related information; and
- determining the one or more hemodynamic parameters of the subject from the obtained tissue pressure information, the estimated fat-free mass and the subject demographic information.

**15.** Computer program comprising program code means for causing a computer to carry out the steps of the method as claimed in claim 14 when said computer program is carried out on the computer.

FIG.1

FIG.2

FIG.3A

FIG.3B

13

Input — Processing — Output

30     31     32

FIG.4

13

ShellCuff system

Tissue pressure

$M_1$
⋮
$M_n$

FFM estimation — FFM — ShellCuff algorithm — HDM parameters

Patient demographics

FIG.5

13

ShellCuff system

Tissue pressure
Actuator pressure

FFM estimation — FFM — ShellCuff algorithm — HDM parameters

Patient demographics

FIG.6

FIG.7A    FIG.7B    FIG.7C

FIG.8A

FIG.8B

FIG.8C

EP 4 413 919 A1

Providing a first PCSV_uncal — S100

Determining perfusion parameter BioCal — S200

Adjusting the first PCSV_uncal based on the perfusion parameter BioCal — S280

Outputting second PCSV_imp — S290

FIG.9

Determining one or more characteristics of an arterial blood pressure waveform — S70

Identifying a portion of the arterial pressure waveform — S80

Determining a first PCSV_uncal based on at least one portion of the arterial pressure waveform — S85

Outputting a first PCSV_uncal — S90

FIG.9A

Providing a first PCSV_uncal

FIG.9B

Determining the
fat free mass FFM ⟋S205

Determining the
adipose mass AM ⟋S255

Determining layer
thickness of fat free
mass FFM ⟋S210

Determining
layer thickness of
adipose mass AM ⟋S260

Determining a perfusion
coefficient B of fat free
mass, B_FFM, based on
FFM layer thickness ⟋S215

Determining a perfusion
coefficient B of adipose
mass, B_AM, based on
AM layer thickness ⟋S265

Determining B_FFM_corr
by applying
AM_B_FFM_Red to B_FFM ⟋S220

Determining a
reducer function
AM_B_FFM_Red ⟋S270

Determining a PCSV
calibration factor
PCSV_FFM_CAL based
on B_FFM_corr and FFM ⟋S225

Determining a PCSV
calibration factor
PCSV_AM_CAL based
on B_AM and AM ⟋S275

Apply demographic calibration factor P_Cal to sum of
PCSV_FFM_CAL and PCSV_AM_CAL ⟋S230

Outputting a perfusion parameter BioCal ⟋S240

FIG.10

25

FIG.11

FIG.12

FIG.13

FIG.14

FIG.15

Europäisches
Patentamt

European
Patent Office

Office européen
des brevets

# EUROPEAN SEARCH REPORT

**Application Number**

EP 23 15 5623

## DOCUMENTS CONSIDERED TO BE RELEVANT

| Category | Citation of document with indication, where appropriate, of relevant passages | Relevant to claim |
|---|---|---|
| X | US 2021/228095 A1 (PFEIFFER ULRICH [DE] ET AL) 29 July 2021 (2021-07-29)<br>* figure 35 *<br>* paragraphs [0098], [0132], [0144], [0282], [0278] * | 1-15 |
| A | US 7 316 652 B2 (BANG & OLUFSEN MEDICOM AS [DK]) 8 January 2008 (2008-01-08)<br>* figure 4 *<br>* column 7, line 1 - line 2 * | 2,3,6,7, 12,13 |
| A | US 2015/359446 A1 (PFEIFFER ULRICH [DE] ET AL) 17 December 2015 (2015-12-17)<br>* figures 1a, b, 2a, b * | 2,3,6,7, 12,13 |

**CLASSIFICATION OF THE APPLICATION (IPC)**

INV.
A61B5/029
A61B5/00

**TECHNICAL FIELDS SEARCHED (IPC)**

A61B
G16H

The present search report has been drawn up for all claims

| Place of search | Date of completion of the search | Examiner |
|---|---|---|
| The Hague | 28 June 2023 | Schwenke, Stephanie |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 15 5623

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2023

| Patent document cited in search report | | Publication date | Patent family member(s) | | Publication date |
|---|---|---|---|---|---|
| US 2021228095 | A1 | 29-07-2021 | CN | 112040850 A | 04-12-2020 |
| | | | EP | 3787488 A1 | 10-03-2021 |
| | | | JP | 2021523764 A | 09-09-2021 |
| | | | US | 2021228095 A1 | 29-07-2021 |
| | | | WO | 2019211210 A1 | 07-11-2019 |
| US 7316652 | B2 | 08-01-2008 | AT | 370700 T | 15-09-2007 |
| | | | BR | 0307095 A | 28-12-2004 |
| | | | CA | 2473472 A1 | 31-07-2003 |
| | | | CN | 1622786 A | 01-06-2005 |
| | | | EP | 1480555 A1 | 01-12-2004 |
| | | | JP | 2005515010 A | 26-05-2005 |
| | | | MX | PA04007016 A | 10-11-2005 |
| | | | US | 2005228302 A1 | 13-10-2005 |
| | | | WO | 03061468 A1 | 31-07-2003 |
| US 2015359446 | A1 | 17-12-2015 | CN | 105228516 A | 06-01-2016 |
| | | | CN | 110811586 A | 21-02-2020 |
| | | | JP | 6360838 B2 | 18-07-2018 |
| | | | JP | 2016509516 A | 31-03-2016 |
| | | | US | 2015359446 A1 | 17-12-2015 |
| | | | US | 2022257130 A1 | 18-08-2022 |
| | | | WO | 2014121805 A1 | 14-08-2014 |
| | | | WO | 2014121945 A1 | 14-08-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015359446 A1

- WO 2019211210 A1

**Non-patent literature cited in the description**

- **JANMAHASATIAN, S. ; DUFFULL, S.B. ; ASH, S. et al.** Quantification of Lean Bodyweight. *Clin Pharmacokinet,* 2005, vol. 44, 1051-1065
- **CHEN et al.** *Comput Cardiol.,* 01 January 2009
- **YU et al.** *BMC Pharmacol Toxicol.,* 14 October 2013

- **RIPKA WL ; ORSSO CE ; HAQQ AM ; PRADO CM ; ULBRICHT L ; LEITE N.** Validity and accuracy of body fat prediction equations using anthropometries measurements in adolescents. *Eat Weight Disord,* April 2021, vol. 26 (3), 879-886
- **COLLIS et al.** *Circulation,* 13 February 2001, vol. 103 (6
- **CORDEN et al.** *J Cardiovasc Magn Reson.,* 31 May 2016, vol. 18 (1), 32